# EUROPÄISCHE PATENTSCHRIFT

(11) **EP 0 842 917 B1**
(45) Veröffentlichungstag und Bekanntmachung des Hinweises auf die Patenterteilung: **19.07.2000**
(21) Anmeldenummer: 97118210.0
(22) Anmeldetag: 21.10.1997
(51) Int. Cl.: C07C 45/67, C07C 49/203

(54) **Verfahren zur Herstellung Gamma,Delta-ungesättigten Ketonen durch Carroll-Reaktion in cyclischen Carbonaten oder Gamma-Lactonen als Lösungsmittel**
Process for the preparation of gamma, delta-unsaturated ketones by Carroll-reaction in cyclic carbonates or gamma-lactones as solvent
Procédé pour la préparation de cétones gamma, delta insaturées par la réaction de Carroll dans des carbonates cycliques ou des gamma-lactones comme solvant

(30) Priorität: 14.11.1996 DE 19647117
(43) Veröffentlichungstag der Anmeldung: 20.05.1998
(73) Patentinhaber: BASF AKTIENGESELLSCHAFT, 67056 Ludwigshafen (DE)
(72) Erfinder: Etzrodt, Heinz, Dr., 67434 Neustadt (DE); Stroezel, Manfred, 68549 Ilvesheim (DE); Weller, Dietmar, Dr., 67067 Ludwigshafen (DE); Jaedicke, Hagen, Dr., 67069 Ludwigshafen (DE)

(56) Entgegenhaltungen:
- EP-A- 0 022 955
- FR-A- 1 205 812
- US-A- 3 860 655

## Beschreibung

Die Erfindung betrifft die Herstellung von γ,δ-ungesättigten Ketonen durch Carroll-Reaktion in besonders vorteilhaften Lösungsmitteln.

γ,δ-ungesättigte Ketone, wie das 2-Methyl-2-hepten-6-on, sind begehrte Zwischenprodukte für die Herstellung von Riechstoffen, Vitamin A, Vitamin E und Pharmazeutika. Es hat daher nie an Versuchen gefehlt, diese Verbindungen auf vorteilhafte Weise herzustellen.

Eine wichtige bekannte Herstellungsmethode ist die Umsetzung von Vinylcarbinolen oder Propargylalkoholen mit Acetessigsäurealkylestern oder Diketen und eine anschließende thermische Umlagerung der erhaltenen Acetoacetate (Carroll-Reaktion; vgl. M.F. Carroll in J. Chem. Soc. 1940, Seite 704 - 706 und 1266 - 1268).

Aus US 2 638 484 ist es bekannt, 2-Methyl-3-buten-2-ol in Gegenwart von metallischem Natrium mit Diketen umzusetzen und das erhaltene Vinyldimethylcarbinolacetoacetat durch vorsichtiges Erhitzen unter Rückfluß in das gewünschte 2-Methyl-2-hepten-6-on umzulagern. Nachteilig an diesem Verfahren ist, daß man den ersten Reaktionsschritt bei Temperaturen von etwa 0°C durchführen muß, daß man nicht kontinuierlich arbeiten kann und daß das Arbeiten mit Natrium und Diketen in technischem Maßstab problematisch ist.

Aus US 2 795 617 ist es bekannt, den zweiten Reaktionsschritt, d.h. die Umlagerung der Acetoacetate in Gegenwart von katalytischen Mengen von Aluminiumtrialkoxiden, wie Aluminiumtriisopropylat, bei Temperaturen von 135 bis 165°C durchzuführen. Die Umsetzung wird in Abwesenheit eines Lösungsmittels durchgeführt. Auch bei diesem Verfahren lassen die Ausbeuten an γ,δ-ungesättigten Ketonen noch zu wünschen übrig.

Gemäß dem Verfahren von GB 695 313 wurde das Acetoacetat von 2-Methyl-3-buten-2-ol in der Gasphase auf 300 bis 600°C erhitzt. Die erzielten Ausbeuten an ungesättigten Ketonen liegen jedoch bei nur 13,8 bis maximal 53,4 %, bezogen auf eingesetztes Acetoacetat. Gemäß dem Verfahren von GB 886 353 erfolgt die Umlagerung der Allylacetoacetate durch deren Erhitzen in Gegenwart von Aluminiumtri(acetylacetonaten) auf 140 bis 170°C. auch bei diesem Verfahren lassen die erzielten Ausbeuten noch zu wünschen übrig.

Um bei der Herstellung der Allylacetoacetate den problematischen Umgang mit Diketen zu vermeiden, setzt man bei Umsetzungen in technischem Maßstab häufig anstelle von Diketen die leicht hieraus herstellbaren niederen Alkylester der Acetessigsäure ein. Bei Temperaturen oberhalb von 100°C spaltet sich aus dem Acetessigsäureester der Alkohol ab und kann abdestilliert werden (vgl. J. Org. Chem. 56 (1991), 1713 - 1718).

Setzt man 2-Methyl-3-buten-2-ol (Kp. 98°C) nach dieser Methode um, so wird die für die anschließende Umlagerung des Allylacetoacetats notwendige Reaktionstemperatur von 120 bis 220 °C bei Normaldruck nur dadurch erreicht, daß man den Acetessigsäureethylester (Kp. 169°C) in großem Überschuß einsetzt (vgl. DBP 1 068 696) unter den Reaktionsbedingungen reagiert jedoch der vorgelegte Acetessigsäureester auch mit sich selber und die unter anderen Nebenprodukten gebildete Dehydrazetsäure löst noch weitere Nebenreaktionen aus.

Es war daher die Aufgabe der Erfindung, das Verfahren zur Herstellung von γ,δ-ungesättigten Ketonen durch Umsetzen von Vinylcarbinolen oder Propargylalkoholen mit Acetessigsäurealkylestern oder Diketen und anschließende thermische Umlagerung der erhaltenen Acetoacetate so zu verbessern, daß man ohne grossen Überschuß an Acetessigsäureestern auskommt, sowie daß man die Umlagerung der Acetoacetate unter Normaldruck kontinuierlich und mit sehr guten Ausbeuten durchführen kann.

Es wurde nun überraschenderweise gefunden, daß die Carroll-Reaktionen in 5-Ring-Lactonen und 5-Ring-Carbonaten wesentlich beschleunigt werden. Die für die Umlagerung notwendigen Reaktionstemperaturen von mehr als 150°C werden in den relativ hochsiedenden 5-Ring-Lactonen und 5-Ring-Carbonaten drucklos erreicht, weil das niedrigsiedende Methylbutenol (Kp. 98°C) mit dem Acetessigester schnell abreagiert und 2-Methyl-2-hepten-6-on bildet. Das aus Acetessigsäuremethylester gebildete Methanol destilliert bei den Reaktionstemperaturen von 120 bis 220°C laufend ab, ohne das als Lösungsmittel verwendete cyclische Lacton oder cyclische Carbonat abzubauen.

Gegenstand der Erfindung ist dementsprechend ein Verfahren zur Herstellung von γ,δ-ungesättigten Ketonen durch
a) Umsetzen von Vinylcarbinolen oder Propargylalkoholen mit Acetessigsäurealkylestern oder Diketen und
b) thermische Umlagerung der erhaltenen Acetoacetate ggf. in Gegenwart von einem Aluminiumkatalysator, zu den γ,δ-ungesättigten Ketonen (Carroll-Reaktion),
das dadurch gekennzeichnet ist, daß man den Reaktionsschritt a) und/oder den Reaktionsschritt b) in einem cyclischen Carbonat der allgemeinen Formel I oder einem γ-Lacton der allgemeinen Formel II 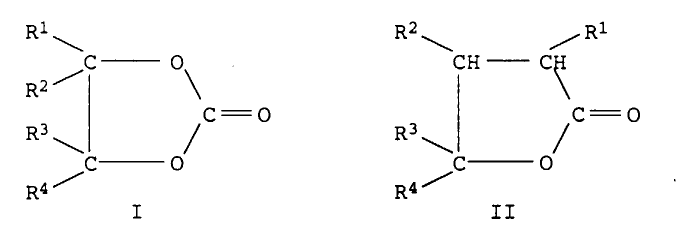 in denen die Reste R¹, R² und R³ für H, Methyl oder Ethyl, vorzugsweise für H oder Methyl stehen und R⁴ für H, Methyl, Ethyl, Isopropyl, Phenyl oder Methoxymethyl steht, als Lösungsmittel durchführt.

Als cyclische 5-Ring-Carbonate der allgemeinen Formel I kommen neben den gebräuchlichen Alkylencarbonaten, wie Ethylencarbonat, 1,2-Propylencarbonat, Isobutylencarbonat und 1,2-Butylen-carbonat, d.h. Carbonaten der allgemeinen Formel I, in der R¹ bis R⁴ für H oder Methyl stehen, bzw. R¹ bis R³ für H oder Methyl und R⁴ Ethyl bedeutet, auch solche in Betracht, in denen R¹ bis R³ zusätzlich für Ethyl stehen kann und R⁴ für H, Methyl, Ethyl, Isopropyl, Phenyl oder Methoxymethyl steht.

Die erfindungsgemäß verwendeten cyclischen Carbonate sind durch Umsetzen der entsprechenden Alkylenoxide mit CO₂ auch großtechnisch äußerst preiswert herstellbar. Sie weisen im allgemeinen so hohe Siedepunkte auf, daß man unter Normaldruck problemlos Temperaturen von 170°C erreichen kann. Sie sind nicht toxisch (Die LD₅₀ für Ratten liegt z.B. für 1,2-Propylencarbonat oral bei 29000 mg/kg und für Ethylencarbonat bei 10000 mg/kg.), was für die Herstellung von Vitaminvorprodukten wesentlich ist, und sind gut biologisch abbaubar (vgl. Firmenschrift "Alkylencarbonate" der Firma Hüls AG, Juli 1991).

Als besonders geeignete 5-Ring-Lactone der allgemeinen Formel II seien γ-Butyrolacton, 3-Methyl-γ-butyrolacton, 3,4-Dimethyl-γ-butyrolacton, 4,5-Dimethyl-γ-butyrolacton und 5-Ethyl-γ-butyrolacton, insbesondere γ-Butyrolacton genannt.

Auch die erfindungsgemäß verwendeten γ-Butyrolactone der allgemeinen Formel II sind großtechnisch vorteilhaft durch Dehydrierung der entsprechenden Butandiole herstellbar.

Das bei der Reaktion gebildete Alkanol greift die cyclischen Carbonate oder Lactone unter den Reaktionsbedingungen überraschenderweise so wenig an, daß beispielsweise bei der Verwendung von Propylencarbonat das Lösungsmittel für bis zu 10 Reaktionszyklen ohne jegliche Aufreinigung wieder verwendet werden kann (vgl. Beispiel 1). Das nach der Isolierung des γ,δ-ungesättigten Ketons abgetrennte cyclische Carbonat oder Lacton kann ohne Ergänzung des Katalysators in neue Reaktionszyklen eingespeist werden. Reste von nicht umgesetztem Acetoacetat bleiben dabei im Lösungsmittel und gehen nicht verloren.

Die 5-Ring-Carbonate und 5-Ring-Lactone verwendet man im allgemeinen in Mengen von 50 bis 1000 Gew.-%, vorzugsweise 100 bis 500 Gew.-%, bezogen auf gebildetes γ,δ-ungesättigtes Keton.

Als Vinylcarbinole oder Propargylalkohole können bei dem erfindungsgemäßen Verfahren Alkohole der allgemeinen Formel III 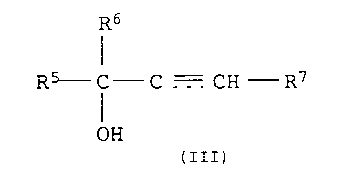 eingesetzt werden, in der
- R⁵: für einen verzweigten oder unverzweigten, gesättigten oder olefinisch ein- oder mehrfach ungesättigten aliphatischen oder cycloaliphatisch-aliphatischen Rest, mit 1 bis 20 C-Atomen, der durch Alkoxygruppen oder Halogen substituiert sein kann, steht,
- R⁶: für H oder eine niedere Alkylgruppe, vorzugsweise eine Methyl- oder Ethylgruppe steht,
- R⁷: für H oder eine niedere Alkylgruppe, vorzugsweise H steht und die gestrichelte Linie eine weitere Bindung bedeuten kann.

Genannt seien beispielsweise 2-Methyl-3-buten-2-ol, Nerolidol, 10,11-Dihydro-nerolidol, Linalool, 6,7-Dihydro-linalool, Geranyllinalool, Isophytol, oder 1-Alkin-3-ole, wie Dehydrolinalool und 2-Methyl-3-butin-2-ol. Mit besonderem Vorteil gelingt die erfindungsgemäße Umsetzung bei Verwendung von 2-Methyl-3-buten-2-ol, also einer Verbindung der Formel III, in der R⁵ und R⁶ Methyl bedeuten und R⁷ für H steht.

Zur Durchführung des erfindungsgemäßen Verfahrens geht man im allgemeinen so vor, daß man im ersten Reaktionsschritt die Allyl- oder Propargylalkohole gemäß dem Stand der Technik, d.h. ohne Lösungsmittel bzw. in einem für die Umsetzung bekannten Lösungsmittel, wie Diethylether, Toluol oder Xylol in Abwesenheit oder in Anwesenheit eines für die Umsetzung bekannten basischen Katalysators, mit Diketen umsetzt und die anschließende thermische Umlagerung der erhaltenen Acetoacetate, gegebenenfalls nach Abdestillieren des eingesetzten Lösungsmittels und gegebenenfalls in Gegenwart von einem Aluminiumkatalysator, in einem 5-Ring-Carbonat der allgemeinen Formel I oder einem γ-Lacton der allgemeinen Formel II als Lösungsmittel durchführt, oder aber, daß man - mit Vorteil - sowohl die Umsetzung der Vinylcarbinole oder Propargylalkohole mit Acetessigsäurealkylestern oder Diketen als auch die anschließende thermische Umlagerung der erhaltenen Acetoacetate , gegebenenfalls in Gegenwart eines Aluminiumkatalysators, in einem 5-Ring-Carbonat der allgemeinen Formel I oder einem γ-Lacton der allgemeinen Formel II als Lösungsmittel durchführt. Die letztgenannte Variante des erfindungsgemäßen Verfahrens hat den großen Vorteil, daß man praktisch in einer Eintopfreaktion arbeiten kann.

Wesentlich für das Verfahren der vorliegenden Erfindung ist also, daß in jedem Fall die zunächst gebildeten Acetoacetate in einem 5-Ring-Carbonat der Formel I oder einem γ-Lacton der Formel II thermisch umgelagert werden.

Die Umsetzung von Allyl- oder Propargylalkoholen mit Diketen wird bekanntermaßen in Gegenwart basischer Katalysatoren durchgeführt. Als solche seien tertiäre Amine, wie Pyridin und insbesondere para-N,N-Dimethylaminopyridin genannt. Führt man sowohl die Umsetzung der Vinylcarbinole oder Propargylalkohole mit Diketen als auch die anschließende thermische Umlagerung in 5-Ring-Carbonaten oder 5-Ring-Lactonen durch, d.h., arbeitet man praktisch in einer Eintopfreaktion, dann empfiehlt es sich, den ersten Reaktionsschritt in Abwesenheit eines basischen Katalysators oder zumindest in Gegenwart nur geringer Mengen davon durchzuführen.

Die Reaktionstemperaturen für die thermische Umlagerung betragen im allgemeinen 120 bis 220°C, vorzugsweise 140 bis 190°C.

Die thermische Umlagerung kann in Abwesenheit oder in Gegenwart eines der bekannten Aluminiumkatalysatoren durchgeführt werden.

Als für die Umsetzung bekannte Aluminiumkatalysatoren kommen beispielsweise Aluminiumalkoholate, wie Aluminium-tri-(isopropoxid) oder eines der Aluminium-tri-(butoxide); oder aber Aluminium-tri-(acetylacetonat) oder Aluminium-tri-(alkylacetylacetate), wie Aluminiumtri-(methylacetylacetat) in Betracht.

Für die für die Vitamin-A- und Vitamin-E-Herstellung wichtige Herstellung von 2-Methyl-2-hepten-6-on durch Umsetzen von 2-Methyl-3-buten-2-ol und Acetessigsäuremethylester ist es besonders vorteilhaft, wenn sowohl die Umsetzung des Methylbutenols mit Acetessigsäuremethylester als auch die thermische Umlagerung der erhaltenen Acetoacetate in einem 5-Ring-Carbonat der Formel I oder einem 5-Ring-Lacton der Formel II durchgeführt werden, d.h. praktisch in einer Eintopfreaktion erfolgen.

Hierbei geht man mit Vorteil so vor, daß man das einen der für diese Umsetzung bekannten Aluminiumkatalysator enthaltende 5-Ring-Carbonat der Formel I oder 5-Ring-Lacton der Formel II vorlegt, auf Temperaturen von etwa 170 bis 180°C erhitzt und in dieses heiße Gemisch langsam eine Mischung aus dem Methylbutenol und dem Acetessigsäuremethylester zudosiert, wobei das gebildete CO₂ entweicht und Niedersieder abdestilliert werden. Nach Beendigung der Umsetzung kann dann das Methylheptenon bei Normaldruck oder vermindertem Druck abdestilliert und der Destillationsrückstand erneut als Reaktionsmedium verwendet werden.

Da auch bei 9-facher Wiederverwendung des erfindungsgemäßen Reaktionsmediums kein signifikanter Abfall der Ausbeute beobachtet wurde, kann man davon ausgehen, daß sich die Umsetzung auch kontinuierlich durchführen läßt.

Mit Hilfe des erfindungsgemäßen Verfahrens lassen sich die insbesondere als Zwischenprodukte für die Herstellung von Riechstoffen, Vitamin A, Vitamin E und Pharmaka begehrten γ,δ-ungesättigten Ketone auch in technischem Maßstab auf einfache Weise und in sehr guten Ausbeuten herstellen.

### Beispiel 1

### Umsetzung von 2-Methyl-3-buten-2-ol mit Acetessigsäuremethylester in 1,2-Propylencarbonat

a) Zu einer Mischung aus 45 g 1,2-Propylencarbonat und 2,8 g eines separat (gemäß GB 886 353) hergestellten Aluminiumtri- (methylacetylacetat) -Katalysators wurde innerhalb von 2 Stunden (h) unter Rühren bei 180°C eine Mischung aus 29,03 g (0,25 mol) Acetessigsäuremethylester (AME; Reinheit 98 %) und 23,68 g (0,275 mol) 2-Methyl-3-buten-2-ol (MBE; Reinheit 94 %) gepumpt. Während dieser Zeit gaste CO₂ aus und destillierten 8 g Leichtsieder ab, die zu etwa 2/3 aus Methanol und etwa 1/3 aus unumgesetztem 2-Methyl-3-buten-2-ol bestanden. Anschließend wurde noch 30 Minuten (min) bei 180°C gerührt, dann abgekühlt und aus dem Reaktionsgemisch bei einem verminderten Druck von 100 mbar das gewünschte 2-Methyl-2-hepten-6-on abdestilliert.
b) Der hierbei angefallene Destillationsrückstand wurde erneut innerhalb von 2 h bei 180°C mit den oben angegebenen Mengen AME und MBE versetzt, das Reaktionsgemisch 30 min bei 180°C gerührt, dann abgekühlt und daraus das erhaltene 2-Methyl-2-hepten-6-on abdestilliert.
c) Vorgang b) wurde noch 8 mal wiederholt. Die mittlere Ausbeute an 2-Methyl-2-hepten-6-on über alle 10 Ansätze betrug 88 % der Theorie, bezogen auf umgesetztes MBE (gaschromatographische Bestimmung mit internem Standard).

### Beispiel 2

a) Herstellung von 10,11-Dihydro-nerolidolacetoacetat aus 10,11-Dihydro-nerolidol und Diketen
   Zu einer Lösung von 9 g (0,04 mol) 10,11-Dihydro-nerolidol und 60 mg para-N,N-Dimethylamino-pyridin in 50 ml Diethylether wurden innerhalb von 20 min bei 20°C 5,05 g (0,074 mol) Diketen getropft. Anschließend wurde das Lösungsmittel schonend bei 40°C abdestilliert.
b) Carroll-Reaktion von 10,11-Dihydro-nerolidolacetoacetat in 1,2-Propylencarbonat.
   Das in Stufe a) erhaltene Reaktionsgemisch wurde ohne weitere Reinigung innerhalb von 20 min bei 180°C in ein Gemisch aus 20 ml 1,2-Propylencarbonat und 300 mg Aluminium-tri-(methylacetylacetat) getropft. Anschließend wurde noch 10 min bei 180°C gerührt und dann 2 x mit je 50 ml Heptan extrahiert. Die vereinigten Extrakte wurden destilliert. Die Ausbeute an 13,14-Dihydro-farnesylaceton betrug 88 % der Theorie, bezogen auf 10,11-Dihydro-nerolidol.
   Auch hierbei kann das den Katalysator enthaltende Propylencarbonat wiederverwendet werden.

### Beispiel 3

a) Umsetzung von 2-Methyl-3-buten-2-ol mit Acetessigsäuremethylester in γ-Butyrolacton.
   Eine Lösung aus 100 ml γ-Butyrolacton und 5,6 g des in Beispiel 1 angegebenen Katalysators wurde auf Temperaturen von 170 bis 180°C erhitzt und hierzu innerhalb von 30 min unter Rühren bei 170 bis 180°C eine Mischung aus 58,06 g (0,5 mol) AME und 47,36 g (0,55 mol) MBE getropft. Anschließend rührte man noch 30 min bei 170°C, kühlte ab und destillierte bei Normaldruck das erhaltene 2-Methyl-2-hepten-6-on ab. Man isolierte so 59,7 g eines 86 Gew.-%igen Methylheptenons.
b) Der in Stufe a) angefallene Destillationsrückstand wurde erneut innerhalb von 30 min bei 170 bis 180°C mit einer Mischung aus 58,06 g AME und 47,36 g MBE versetzt, das Reaktionsgemisch noch 30 min bei 170°C gerührt, abgekühlt und daraus das Methylheptenon abdestilliert.
c) Der Vorgang b) wurde noch 3 mal wiederholt. Die mittlere Ausbeute an 2-Methyl-2-hepten-6-on über alls 5 Ansätze betrug 85 % der Theorie, bezogen auf eingesetztes MBE.

### Beispiel 4

### Umsetzung von 2-Methyl-3-butin-2-ol mit Acetessigsäuremethylester in 1,2-Propylencarbonat.

Eine Mischung aus 5,3 g des in Beispiel 1 genannten Aluminiumtri-(methylacetylacetat)-Katalysators und 85 g 1,2-Propylencarbonat wurde auf 170°C erwärmt und hierzu bei dieser Temperatur innerhalb von 4 h gleichmäßig eine Mischung aus 55 g (0,47 mol) Acetessigsäuremethylester und 43,7 g (0,52 mol)
2-Methyl-3-butin-2-ol zudosiert. Nach Zulaufende wurde noch 30 min bei 170°C gerührt. Während dieser Zeit gaste CO₂ aus und destillierten Leichtsieder ab. Anschließend wurde abgekühlt und aus dem Reaktionsgemisch bei einem verminderten Druck von ca.
100 mbar das gebildete 2-Methyl-2,4-heptadien-6-on abdestilliert. Es wurden so 42 g reines 2-Methyl-2,4-heptadien-6-on erhalten, was einer Ausbeute von 72 % der Theorie bezogen auf umgesetztes 2-Methyl-3-butin-2-ol entspricht.

## Patentansprüche

1. Verfahren zur Herstellung von γ,δ-ungesättigten Ketonen durch
a) Umsetzen von Vinylcarbinolen oder Propargylalkoholen mit Acetessigsäurealkylestern oder Diketen und
b) thermische Umlagerung der erhaltenen Acetoacetate ggf. in Gegenwart von einem Aluminiumkatalysator, zu γ,δ-ungesättigten Ketonen (Carroll-Reaktion),
dadurch gekennzeichnet, daß man den Reaktionsschritt a) und/oder den Reaktionsschritt b) in einem cyclischen Carbonat der allgemeinen Formel I oder einem γ-Lacton der allgemeinen Formel II 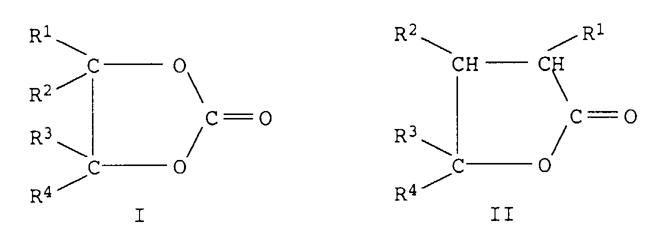 in denen die Reste R¹, R² und R³ für H, Methyl oder Ethyl, vorzugsweise für H oder Methyl stehen und R⁴ für H, Methyl, Ethyl, Isopropyl, Phenyl oder Methoxymethyl steht, als Lösungsmittel durchführt.

2. Verfahren zur Herstellung von γ,δ-ungesättigten Ketonen gemäß Anspruch 1, dadurch gekennzeichnet, daß man den Reaktionsschritt a) und/oder den Reaktionsschritt b) in Ethylencarbonat, 1,2-Propylencarbonat, 1,2-Butylencarbonat oder Isobutylencarbonat als Lösungsmittel durchführt.

3. Verfahren zur Herstellung von γ,δ-ungesättigten Ketonen gemäß Anspruch 1, dadurch gekennzeichnet, daß man den Reaktionsschritt a) und/oder den Reaktionsschritt b) in γ-Butyrolacton, 3-Methyl-γ-butyrolacton, 3,4-Dimethyl-γ-butyrolacton, 4,5-Dimethyl-γ-butyrolacton oder 5-Ethyl-γ-butyrolacton als Lösungsmittel durchführt.

4. Verfahren gemäß Anspruch 1, dadurch gekennzeichnet, daß man den Reaktionsschritt b) bei Temperaturen von 120 bis 220°C, vorzugsweise 150 bis 200°C durchführt.

5. Verfahren gemäß Anspruch 1, dadurch gekennzeichnet, daß man zur Herstellung von 2-Methyl-2-hepten-6-on ein Gemisch von 2-Methyl-3-buten-2-ol und Acetessigsäuremethylester bei Temperaturen von 170 bis 180°C langsam in ein Gemisch aus einem cyclischen Carbonat der allgemeinen Formel I oder einem γ-Lacton der allgemeinen Formel II und einem Aluminiumkatalysator zudosiert, nach Beendigung der Umsetzung das Methylheptenon abdestilliert und den Destillationsrückstand erneut als Reaktionsmedium verwendet.

## Claims

1. A process for preparing γ,δ-unsaturated ketones by
a) reacting vinylcarbinols or propargyl alcohols with alkyl acetoacetates or diketene and
b) thermally rearranging the resulting acetoacetates in the presence or absence of an aluminum catalyst to γ,δ-unsaturated ketones (Carroll reaction),
wherein step a) and/or step b) is carried out in a cyclic carbonate of the formula I or a γ-lactone of the formula II 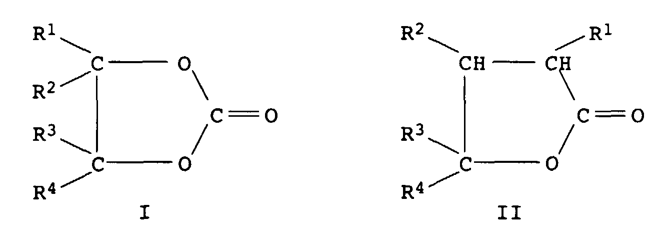 where R¹, R² and R³ are H, methyl or ethyl, preferably H or methyl, and R⁴ is H, methyl, ethyl, isopropyl, phenyl or methoxymethyl, as solvent.

2. A process for preparing γ,δ-unsaturated ketones as claimed in claim 1, wherein step a) and/or step b) is carried out in ethylene carbonate, 1,2-propylene carbonate, 1,2-butylene carbonate or isobutylene carbonate as solvent.

3. A process for preparing γ,δ-unsaturated ketones as claimed in claim 1, wherein step a) and/or step b) is carried out in γ-butyrolactone, 3-methyl-γ-butyrolactone, 3,4-dimethyl-γ-butyrolactone, 4,5-dimethyl-γ-butyrolactone or 5-ethyl-γ-butyrolactone as solvent.

4. A process as claimed in claim 1, wherein step b) is carried out at from 120 to 220°C, preferably 150 to 200°C.

5. A process as claimed in claim 1, wherein
2-methyl-2-hepten-6-one is prepared by slowly metering a mixture of 2-methyl-3-buten-2-ol and methyl acetoacetate at from 170 to 180°C into a mixture of a cyclic carbonate of the formula I or a γ-lactone of the formula II and an aluminum catalyst, distilling off the methylheptenone after the reaction is complete, and using the distillation residue anew as reaction medium.

## Revendications

1. Procédé pour la préparation de cétones γ,δ-insaturées par
a) réaction de vinylcarbinols ou d'alcools propargyliques avec des acétylacétates d'alkyle ou le dicétène et
b) transposition à la chaleur des acétylacétates obtenus, éventuellement en présence d'un catalyseur à base d'aluminium, en les cétones γ,δ-insaturées (réaction de Carroll),
caractérisé par le fait que, au stade de réaction a) et/ou au stade de réaction b), on opère dans un solvant consistant en un carbonate cyclique de formule générale I ou en une gamma-lactone de formule générale II 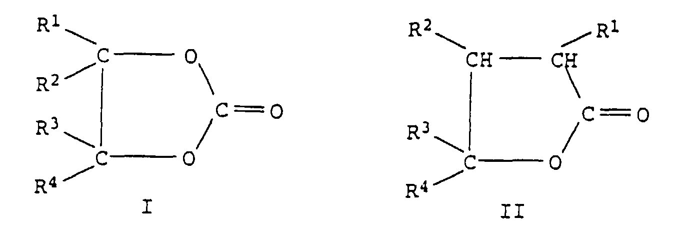 dans lesquelles les symboles R¹, R² et R³ représentent H, des groupes méthyle ou éthyle, de préférence H ou des groupes méthyle et R⁴ représente H, un groupe méthyle, éthyle, isopropyle, phényle ou méthoxyméthyle.

2. Procédé pour la préparation de cétones γ,δ-insaturées selon la revendication 1, caractérisé par le fait que, au stade de réaction a) et/ou au stade de réaction b), on opère dans un solvant consistant en le carbonate d'éthylène, le carbonate de 1,2-propylène, le carbonate de 1,2-butylène ou le carbonate d'isobutylène.

3. Procédé pour la préparation des cétones γ,δ-insaturées selon la revendication 1, caractérisé par le fait que, au stade de réaction a) et/ou au stade de réaction b), on opère dans un solvant consistant en la gamma-butyrolactone, la 3-méthyl-gamma-butyrolactone, la 3,4-diméthyl-gamma-butyrolactone, la 4,5-diméthyl-gamma-butyrolactone ou la 5- éthylgamma-butyrolactone.

4. Procédé selon la revendication 1, caractérisé par le fait que, au stade de réaction b), on opère à des températures de 120 à 220°C, de préférence de 150 à 200°C.

5. Procédé selon la revendication 1, caractérisé par le fait que, pour la préparation de la 2-méthyl-2-heptén-6-one, on ajoute un mélange de 2-méthyl-3-butén-2-ol et d'acétylacétate de méthyle lentement, à des températures de 170 à 180°C, à un mélange d'un carbonate cyclique de formule générale I ou d'une gamma-lactone de formule générale II et d'un catalyseur à base d'aluminium, et lorsque la réaction des terminée, on distille la méthyl-hepténone et on utilise à nouveau le résidu de distillation en tant que milieu de réaction.
